# EUROPÄISCHE PATENTSCHRIFT

(11) **EP 3 821 972 B1**
(45) Veröffentlichungstag und Bekanntmachung des Hinweises auf die Patenterteilung: **10.07.2024**
(21) Anmeldenummer: 19208713.8
(22) Anmeldetag: 12.11.2019
(51) Int. Cl.: B01D 63/02, A61K 31/00, B01D 61/18

(54) **VERWENDUNG EINES FILTERMODULS ZUR FILTRATION EINER BIOTECHNOLOGISCHEN FLÜSSIGKEIT UND FILTERMODUL ZUR FILTRATION EINER BIOTECHNOLOGISCHEN FLÜSSIGKEIT**
FILTER MODULE FOR FILTERING A BIOTECHNOLOGICAL FLUID AND USE OF A FILTER MODULE FOR FILTERING A BIOTECHNOLOGICAL FLUID
UTILISATION D'UN MODULE FILTRANT POUR FILTRER UN LIQUIDE BIOTECHNOLOGIQUE ET MODULE FILTRANT PERMETTANT DE FILTRER UN LIQUIDE BIOTECHNOLOGIQUE

(43) Veröffentlichungstag der Anmeldung: 19.05.2021
(73) Patentinhaber: Fresenius Medical Care Deutschland GmbH, 61352 Bad Homburg (DE); Unicyte EV AG, 6370 Oberdorf Nidwalden (NW) (CH)
(72) Erfinder: Heide, Herr Alexander, 65817 Eppstein (DE); Nikolic, Herr Dejan, 65812 Bad Soden (DE)
(74) Vertreter: Dreyhsig, Jörg

(56) Entgegenhaltungen:
- EP-A1- 0 576 677
- EP-A1- 1 398 047
- EP-B1- 0 576 677
- EP-B1- 1 398 047
- US-A- 5 263 924
- US-A- 5 830 370
- US-B1- 8 647 569
- US-B2- 7 871 566

## Beschreibung

Die vorliegende Erfindung betrifft die Verwendung eines Filtermoduls sowie ein Filtermodul zur Filtration einer biotechnologischen Flüssigkeit und eine Filtrationsvorrichtung mit einem solchen Filtermodul.

Aus dem Stand der Technik sind biotechnologische Verarbeitungsverfahren biotechnologischer Flüssigkeiten bekannt, wie beispielsweise biotechnologische Flüssigkeiten durch ein TFF ("tangential flow filtration") Verfahren zu reinigen bzw. aufzubereiten. Hierbei wird ähnlich wie bei einer Dialyse eine Substratflüssigkeit in einem Kreislauf geführt und dabei mittels einer Pumpe mehrfach durch ein Hohlfasermembranmodul geleitet. Dabei treten ein Teil der Flüssigkeit sowie kleine Moleküle durch die Membran hindurch auf die Permeatseite über. Permeat nennt man die durch die Membrane hindurchtretende Substanz. Die Permeatseite ist die Außenseite einer Hohlfaser, wenn die primäre biotechnologische Flüssigkeit in den Innenraum der Hohlfaser geleitet wird. Dadurch verringert sich das Volumen des Teils der Flüssigkeit, der im Kreislauf verbleibt und nicht durch die Membrane der Hohlfasern hindurchtritt. Diesen Teil der Flüssigkeit nennt man Retentat. Die Verringerung des Volumens des Retentats bedingt also, dass die aufzureinigenden biotechnologischen Materialien in dem verringerten Volumen, bereinigt um die kleinen Moleküle, in dem Retentat auf der Retentatseite verbleiben. In anderen Worten wird die Konzentration der erwünschten Bestandteile im Retentat erhöht, indem Permeat abgeschieden wird.

Ein derartiges TFF-Verfahren, das in bevorzugter Ausgestaltung Gegenstand der vorliegenden Erfindung ist, ist beispielsweise aus der WO 2017/117585 A1 bekannt.

Weiterhin sind Zentrifugalpumpen und Pumpvorrichtungen mit Zentrifugalpumpen bekannt, welche rotationsfähige Bestandteile im Inneren eines Pumpenkopfes aufweisen, durch deren Rotation ein zu förderndes Medium mitgerissen und somit auch in Rotation versetzt wird. Durch die Rotationsbewegung wirken Zentripetalkräfte, welche sich in einem rotierenden Bezugs-system als Zentrifugalkräfte äußern, auf das mitgerissene, zu fördernde Medium. Wenn das zu fördernde Medium nahe der Rotationsachse in das Innere einer derartigen Zentrifugalpumpe gelangt, wird es durch die Zentrifugalkräfte infolge der Rotation nach außen gedrückt und dadurch gefördert, mit anderen Worten gepumpt.

In bekannten biotechnologischen Filtrationsverfahren und Filtrationsvorrichtungen besteht das Problem, dass beim Realisieren eines Flussplans oder Flusskreislaufs zur Filtration eine Pumpe und ein Filter zusammengeschaltet werden und dabei einerseits ein Totraum, beziehungsweise Kanten entstehen, in welchen Verwirbelungen auftreten, welche sich negativ auf den Strömungsverlauf auswirken, und Gefahrenstellen für Kontamination darstellen. Üblicherweise ist eine mechanische Schnittstelle zum Anschluss einer Pumpe an einen Filter erforderlich. Dazu wird üblicherweise ein Flansch genutzt. Auch bekannt sind Vorrichtungen, in welchen zusätzlich ein Leitungsabschnitt zwischen Zentrifugalpumpenrotor und Filter liegt. Bei derartigen Verbindungen liegen mindestens zwei Schnittstellen wie z.B. Flansche zwischen Zentrifugalpumpenrotor und Filter mit oben genannten Nachteilen vor. Mit anderen Worten bedingt dies sogar zwei Eintrittspforten für Kontamination, entsprechend eine verringerte Hygiene und noch mehr Totraum für Flüssigkeit. Die Möglichkeit, magnetisch angetriebene Impeller innerhalb eines Filtergehäuses vorzusehen, ist bekannt aus US 5 263 924 A, US 8 647 569 B1, EP 0 576 677 A1, US 5 830 370 A, US 7 871 566 B2.

Daher ist es Aufgabe der vorliegenden Erfindung, Möglichkeiten zur biotechnologischen Filtration zu schaffen, bei welchen die Gefahr von Kontamination gering ist und gleichzeitig ein optimaler Strömungsverlauf erreicht werden kann.

Diese Aufgabe wird gelöst durch ein Filtermodul zur Filtration einer biotechnologischen Flüssigkeit gemäß Anspruch 1 sowie eine Filtrationsvorrichtung nach Anspruch 9. Außerdem wird diese Aufgabe gemäß Anspruch 10 durch die Verwendung eines Filtermoduls nach Anspruch 1, das in einem Filtergehäuse einen Filter mit einem Bündel Hohlfasern und einen Zentrifugalpumpenrotor aufweist, gelöst.

Es wird die Verwendung eines Filtermoduls, das in einem Filtergehäuse einen Filter mit einem Bündel hohler Fasern und einen Zentrifugalpumpenrotor aufweist, für ein biotechnologisches Produktionsverfahren offenbart.

Weiterhin wird ein Filtermodul zur Filtration einer biotechnologischen Flüssigkeit, welches ein Gehäuse, einen in dem Gehäuse angeordneten Filter mit einem Bündel hohler Fasern, einen Zentrifugalpumpenrotor so angeordnet in dem Gehäuse, dass er fluidisch verbunden ist mit dem Innenraum der hohlen Fasern, wobei der Zentrifugalpumpenrotor magnetisch antreibbar ist, sodass er eine Flüssigkeit durch die hohlen Fasern drücken kann, einen ersten Anschluss für die Zufuhr einer zu filtrierenden biotechnologischen Flüssigkeit, welcher fluidisch verbunden ist mit dem Innenraum der hohlen Fasern des Filters, einen zweiten Anschluss für die Abfuhr einer zu filtrierenden biotechnologischen Flüssigkeit, welcher fluidisch verbunden ist mit dem Innenraum der hohlen Fasern des Filters und einen dritten Anschluss zur Abfuhr von abgeschiedener Abfallflüssigkeit, welche fluidisch verbunden ist mit dem Außenraum der hohlen Fasern aufweist, offenbart.

Ein solches Filtermodul kann gemäß einem Aspekt der Erfindung insbesondere für ein biotechnologisches Filtrationsverfahren, insbesondere ein Tangentialfluss-Filtrationsverfahren (TFF), ein Verfahren zur Filtration oder Konzentration extrazellulärer Vesikel in einer biotechnologischen Flüssigkeit oder ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit eingesetzt werden.

Diese Verwendung eines Filtermoduls, das in einem Filtergehäuse einen Filter mit einem Bündel hohler Fasern und einen Zentrifugalpumpenrotor aufweist, für ein biotechnologisches Produktionsverfahren und das Filtermodul zur Filtration einer biotechnologischen Flüssigkeit, welches ein Gehäuse mit einem darin angeordneten Zentrifugalpumpenrotor und einen im Gehäuse angeordneten Filter mit einem Bündel hohler Fasern aufweist ermöglicht besonders vorteilhaft eine gesteigerte Hygiene und eine verringerte Kontamination, weil keine mechanische Schnittstelle zum Anschluss zwischen Zentrifugalpumpenrotor und Filter besteht.

Bevorzugt sind die Fasern des Faserbündels des Filters bei der erfindungsgemäßen Verwendung vertikal zur Erdoberfläche, d.h. parallel zur Richtung der lokalen Erdanziehungskraft, ausgerichtet und es ist der Zentrifugalpumpenrotor auf der Längsachse des Bündels koaxial unterhalb des Bündels angeordnet, sodass im Betrieb der Pumpe die Flüssigkeit nach oben strömt. Eine vertikale Anordnung von Zentrifugalpumpenrotor und Filterfasern ergibt den Vorteil, dass eventuell in der Flüssigkeit vorhandene Gasansammlungen durch den Auftrieb und zugleich die von der Pumpe erzeugte Flüssigkeitsströmung nach oben abtransportiert werden. Solche Gasansammlungen sind unerwünscht. Bevorzugt sind die Fasern des Filters parallel zur Rotationsachse des Zentrifugalpumpenrotors angeordnet. Dabei ist der Zentrifugalpumpenrotor stromab des Filters angeordnet. Der Filter befindet sich bevorzugt in Bezug zu einer Standebene der Vorrichtung oberhalbe des Zentrifugalpumpenrotors. Mit anderen Worten wird Flüssigkeit von dem Zentrifugalpumpenrotor entgegen der Schwerkraftrichtung zu dem Filter gepumpt. Dies hat insbesondere den Vorteil, dass Gasblasen unterstützt durch die Strömungsrichtung des Fluids, nach oben, entgegen der Schwerkraft, aufsteigen.

Bevorzugt ist der Zentrifugalpumpenrotor ein magnetisch levitierend lagerbarer und magnetisch antreibbarer Impellerrotor. Bei einer derartigen Ausführung sind die Lagerung und der Antrieb des Zentrifugalpumpenrotors rein magnetisch. Es entfallen also mechanische Lager. Daher entsteht auch keine lokale Hitzequelle, wie es bei einer mechanischen Lagerung eines Zentrifugalpumpenrotors mit einer Welle oder einer Achse aufgrund von Reibungskräften der Fall wäre. Daraus entsteht der Vorteil einer verringerten Schädigung der biotechnologischen Flüssigkeit, die mit dem Zentrifugalpumpenrotor in Kontakt kommt. Denn Hitze ist für die allermeisten biotechnologischen Flüssigkeiten schädlich.

In einem erfindungsgemäßen Filter ist das Bündel von Fasern so angeordnet, dass die Fasern parallel zueinander verlaufen. Die Fasern können eine Wellung aufweisen oder gerade sein. Auch wenn sie eine Wellung aufweisen, verlaufen sie makroskopisch betrachtet etwa gerade. Eine Längsachse eines Filters verläuft parallel zu den Fasern des Bündels. Wenn das Bündel einer zylindrischen Anordnung von Fasern entspricht, ist die Längsachse mit der Rotationsachse des Zylinders gleichzusetzen. Ein Filtergehäuse einer erfindungsgemäßen Filtrationsvorrichtung ist entsprechend länglich und seine Längsrichtung erstreckt sich entlang der Längsachse eines solchen Bündels.

Jede hohle Faser (auch Hohlfaser) eines Faserbündels definiert einen Hohlraum in ihrem Inneren. Zwischen dem Hohlraum im Inneren einer Faser und dem Außenraum einer Faser liegt die Wand der Faser. Erfindungsgemäß ist die Wand eine Membran. Bevorzugt ist die Membran porös. Ist ein Filter aus einem Bündel hohler Fasern gebildet, so bilden die Membrane der Fasern die Membran des Filters.

Gemäß einem Aspekt der vorliegenden Anmeldung im Hinblick auf die Verwendung eines Filtermoduls, das in einem Filtergehäuse einen Filter mit einem Bündel hohler Fasern und einen Zentrifugalpumpenrotor aufweist, für ein biotechnologisches Produktionsverfahren sowie im Hinblick auf ein Filtermodul zur Filtration einer biotechnologischen Flüssigkeit oder eine Filtrationsvorrichtung weist ein Filter des Filtermoduls bevorzugt eine Ausschlussgrenze (Englisch auch molecular weight cut off (MWCO)) von 100-1000 kDa auf. Eine Ausschlussgrenze in diesem Bereich ermöglicht beispielsweise in besonders vorteilhafter Weise biotechnologische Produktionsverfahren oder biotechnologische Filtrationsverfahren.

In anderen Worten weist in diesem bevorzugten Fall die Membran des Filters eine Ausschlussgrenze in dem Bereich100-1000 kDa auf.

Im Kontext dieser Anmeldung ist die Verwendung eines Filtermoduls, ein Filtermodul und eine Filtrationsvorrichtung in Bezug auf biologische Flüssigkeiten sowie biotechnologische Flüssigkeiten zu verstehen. Biotechnisch ist als Kurzform von biotechnologisch zu sehen und somit gleichbedeutend. Da keine Abgrenzung zwischen biologischer Flüssigkeit und biotechnologischer Flüssigkeit allgemein etabliert ist, wird festgestellt, dass beides Flüssigkeiten mit biologischen Bestandteilen sind. Biotechnologisch ist dabei eine Kurzform gleichbedeutend mit biotechnologisch.

Für die Verwendung eines Filtermoduls aufweisend, ein Filtergehäuse, einen Filter mit einem Bündel hohler Fasern und einen Zentrifugalpumpenrotor, für ein biotechnologisches Produktionsverfahren und für ein Filtermodul zur Filtration einer biotechnologischen Flüssigkeit aufweisend ein Gehäuse, einen Filter mit einem Bündel hohler Fasern und einen Zentrifugalpumpenrotor angeordnet im Gehäuse ist es im Kontext der vorliegenden Anmeldung wesentlich, dass es mindestens drei Anschlüsse am Filtergehäuse gibt, durch die die biotechnologische Flüssigkeit oder die abgeschiedene Abfallflüssigkeit geführt wird. Mindestens zwei Anschlüsse sind fluidisch mit dem Innenraum der Hohlfasern des Faserbündels des Filters verbunden und mindestens ein Anschluss ist fluidisch vom Innenraum der Hohlfasern des Faserbündels des Filters getrennt aber zugleich fluidisch mit dem Außenraum der Hohlfasern des Faserbündels verbunden. Dabei sind die mindestens zwei mit dem Innenraum verbundenen Anschlüsse so angeordnet, dass sie an den gegenüberliegenden Enden der Fasern angeordnet sind. In anderen Worten: Wenn das Faserbündel in einer Richtung von einer Flüssigkeit durchströmt wird, liegt der eine mit dem Innenraum verbundene Anschluss stromaufwärts des Faserbündels und der andere mit dem Innenraum verbundene Anschluss liegt stromabwärts des Faserbündels. Das ist erforderlich, da die beiden mit dem Innenraum der Hohlfasern verbundenen Anschlüsse dazu dienen, das Fließen einer Flüssigkeit entlang der Fasern durch den Innenraum zu ermöglichen. Der dritte Anschluss ist mit dem Außenraum der Fasern fluidisch verbunden, nicht aber mit dem Innenraum. Er dient dazu, Flüssigkeit aus dem Außenraum der Fasern abzuführen. Bei der Verwendung für ein biotechnologisches Produktionsverfahren wird die durch die Fasern hindurch getretene Flüssigkeit üblicherweise als Abfall angesehen. Diese Flüssigkeit will man aus der biotechnologischen Flüssigkeit entfernen und diese dadurch konzentrieren. Die durch die Fasern getretene Flüssigkeit wird auch als Permeat bezeichnet. Das Permeat kann durch einen dritten Anschluss, der am Filtergehäuse angeordnet ist, abgeführt werden. Dieser dritte Anschluss kann an einer ersten Endkappe, an einer zweiten Endkappe oder an einem Mittelteil angeordnet sein. Wesentlich für diesen dritten Anschluss ist, dass er fluidisch verbunden ist mit dem Außenraum der Fasern des Faserbündels des Filters und dass er zugleich fluidisch nicht direkt und vollständig verbunden ist mit dem Innenraum der hohlen Fasern des Faserbündels, sondern durch die Wand der Hohlfasern, die Membran, getrennt ist vom Innenraum der Hohlfasern des Faserbündels.

Bevorzugt ist der Zentrifugalpumpenrotor ein Impeller. Das heißt, dass die Zentrifugalpumpe mit einem radial pumpendem Pumpenrad ausgestattet ist und dieses Pumpenrad ist als Impeller ausgestaltet. Das Pumpenrad wird auch Laufrad der Pumpe genannt. Bevorzugt kann ein Impeller mit hohlem Zentrum gestaltet sein. Beispielsweise kann ein solcher Impeller im Wesentlichen scheibenförmig sein und im Wesentlichen radial verlaufende Schaufelblätter aufweisen, welche ein zu förderndes Pumpmedium bei einer Drehbewegung des Pumpenrads mitnehmen. Das heißt, dass eine erste Scheibe eine Basis formt, auf der Schaufelblätter angeordnet sind. In anderen Worten stehen die Schaufelblätter auf der ersten Scheibe. Das von der ersten Scheibenebene entfernte Ende der Schaufelblätter kann wiederum mit einer weiteren, parallel zur ersten Scheibe angeordneten Scheibe verbunden sein. Optional kann also eine zweite Scheibe die der ersten Scheibe gegenüberliegenden Enden der Schaufelblätter verbinden, sozusagen einen Abschluss bilden. Die zweite Scheibe ist dann parallel zur ersten Scheibe und die Schaufelblätter liegen zwischen den beiden Scheiben. Eine Ausführung mit zwei parallel zueinander angeordneten Scheiben ist ebenso denkbar wie eine Ausführung mit nur einer Scheibe, an der die Schaufelblätter angeordnet sind, währenddessen das scheibenferne Ende der Schaufelblätter frei ist. Eine erste Variante einer solchen Scheibe weist eine zentrale Ausnehmung auf, ist also grob gesagt ringförmig. Eine alternative Variante einer solchen Scheibe ist als Kreisscheibe ausgeführt, weist also gerade keine zentrale Ausnehmung auf.

Im Kontext der vorliegenden Anmeldung ist unter einem Impeller mit hohlem Zentrum zu verstehen, dass die Schaufelblätter sich zum Zentrum hin nicht bis zur Rotationsachse erstrecken, sondern dass ein makroskopisch erkennbarer Bereich an und / oder unmittelbar um die Rotationsachse des Impellers herum frei von Schaufelblättern ist. Das hohle Zentrum ist aber auch nicht mit einem festen Volumenkörper, etwa einem massiven Zylinder, ausgefüllt. Vielmehr ist das Zentrum hohl, sodass die zu pumpende Flüssigkeit durch das hohle Zentrum strömen kann. Es ist denkbar, dass ein massiver Körper genau auf der Rotationsachse des Pumpenrads liegt, z.B. eine Achse oder eine Welle. Dann ist das hohle Zentrum der zentral um die Rotationsachse liegende Bereich zwischen dem massiven Körper und den Schaufelblättern - also der Bereich, den die zu pumpende Flüssigkeit frei durchströmen kann. Das hohle Zentrum kann dann beispielsweise im Wesentlichen wie ein Zylindermantel mit endlicher Manteldicke gestaltet sein.

In einer bevorzugten Ausführungsform weist das Gehäuse des Filtermoduls eine innere Stützplatte mit Öffnungen auf, die zwischen dem Zentrifugalpumpenrotor und dem Filterbündel so angeordnet sind, dass Flüssigkeit vom Zentrifugalpumpenrotor durch die Öffnungen und dann zu den Fasern des Filters strömt, wenn der Zentrifugalpumpenrotor in Drehung versetzt ist.

Gemäß einem Aspekt der vorliegenden Anmeldung sind Zentrifugalpumpenrotor und Faserbündel eines Filtermoduls koaxial angeordnet. Das ermöglicht eine besonders symmetrische Strömung einer Flüssigkeit durch den Zentrifugalpumpenrotor in das Innere der Hohlfasern des Faserbündels. Dann existieren abschnittsweise Antiparallele, koaxiale Flüssigkeitsströme in einer solchen Situation. Die Flüssigkeit strömt den Zentrifugalpumpenrotor auf der Längsachse des Faserbündels, welche mit der Drehachse des Zentrifugalpumpenrotors zusammenfällt, an und wird nach Verlassen des Zentrifugalpumpenrotors so umgelenkt, dass sie parallel jedoch entgegengesetzt gerichtet zum Faserbündel strömt. Bevorzugt ist das Faserbündel makroskopisch genähert zylinderförmig. Durch diese symmetrische Strömungslage wird der Zentrifugalpumpenrotor hydraulisch stabilisiert. Das minimiert ein potentielles Flattern des Zentrifugalpumpenrotors. Ein Flattern des Zentrifugalpumpenrotors könnte zu einer Schädigung der biotechnologischen Flüssigkeit führen. Somit wird auch eine mögliche Schädigung der biotechnologischen Flüssigkeit vermieden. Besonders bei magnetisch levitierend gelagerten Zentrifugalpumpenrotoren ergibt sich gemäß einem weiteren Aspekt der vorliegenden Anmeldung weiterhin der Vorteil, dass weniger exzentrische Lagermomente auftreten oder die exzentrischen Lagermomente sind weniger stark. Dadurch reichen weniger starke Magnetfelder zum Antrieb und zur Lagerung des Zentrifugalpumpenrotors aus. Die weniger starken Magnetfelder ermöglichen wiederum, dass weniger Energie und/oder geringere elektrische Ströme im Erzeuger der magnetischen Wechselfelder der Antriebseinheit benötigt werden.

Im Kontext der vorliegenden Anmeldung kann man betreffend ein Filtermodul oder eine Filtrationsvorrichtung mit einer Filtermodul, wobei ein magnetisch levitierend gelagerter Zentrifugalpumpenrotor in dem Filtergehäuse angeordnet ist, es so bezeichnen, dass nur dann eine voll funktionsfähige Zentrifugalpumpe gebildet ist, wenn das Filtermodul an die Filtrationsvorrichtung so gekoppelt ist, dass die Antriebseinheit der Filtrationseinrichtung mittels dynamischer Magnetfelder den Zentrifugalpumpenrotor antreiben und/oder magnetisch levitierend lagern kann. In anderen Worten: Sofern nicht Antriebseinheit und Filtermodul gekoppelt sind, ist keine funktionstüchtige Pumpe gebildet. Der Zentrifugalpumpenrotor allein ist keine vollständige, funktionstüchtige Pumpe. Ebenso ist die Antriebseinheit einer Filtrationsvorrichtung ohne einen Zentrifugalpumpenrotor keine vollständige, funktionstüchtige Pumpe.

Bei dem biotechnologischen Produktionsverfahren kann es sich um eine Filtration handeln. Bei einer Filtration mit einem biotechnologischen Verfahren wird z.B. eine biotechnologische Flüssigkeit filtriert, gereinigt bzw. aufbereitet.

Im Kontext der vorliegenden Anmeldung ist unter einer biotechnologischen Flüssigkeit auch eine biologische Flüssigkeit zu verstehen. So kann beispielsweise eine biologische Flüssigkeit durch ein biotechnologisches Verfahren zur biotechnologischen Flüssigkeit werden. Daher sind im Kontext dieser Anmeldung, wenn von der Verwendung eines Filtermoduls für ein biotechnologisches Verfahren die Rede ist, Verfahren zu verstehen, bei denen eine Ausgangsflüssigkeit eines Verfahrens eine biotechnologische Flüssigkeit oder eine biologische Flüssigkeit ist. Analog ist im Kontext dieser Anmeldung der Begriff "Filtermodul zur Filtration einer biotechnologischen Flüssigkeit" so zu verstehen, dass auch ein "Filtermodul zur Filtration einer biologischen Flüssigkeit" gemeint ist. Wird eine Flüssigkeit gefiltert, so tritt ein Teil der Flüssigkeitsbestandteile durch einen Filter hindurch. Folglich wird dadurch der Teil der Bestandteile der Flüssigkeit, der nicht durch den Filter hindurchtritt, konzentriert. Je nachdem, welche Bestandteile einer Flüssigkeit wünschenswert sind, entspricht ein solches Verfahren auch einer Reinigung der Flüssigkeitsbestandteile. Bei der zu filternden, zu konzentrierenden, zu reinigenden oder aufzubereitenden biologischen oder biotechnologischen Flüssigkeit handelt es sich beispielsweise um eine Flüssigkeit, die Zellüberstände, Blutkomponenten, wie Blutserum oder -plasma oder Urin ist oder enthält.

Ein Aspekt der vorliegenden Anmeldung betrifft ein Verfahren zur Filtration oder Konzentration biologischer Makromoleküle und biologischer Mikrostrukturen. Bei den zu reinigenden, zu konzentrierenden, zu filtrierenden, in den biotechnologischen Flüssigkeiten vorhandenen biologischen Materialien handelt es sich um biologische Makromoleküle und biologischer Mikrostrukturen. Insbesondere kann es sich dabei um eine oder mehrere der folgenden Substanzen handeln: Antikörper, Antikörper-Konjugate, Antikörperfragment-Konjugate, Viruspartikel, Ribonukleinsäure (kurz RNA für Englisch "ribonucleic acid"), Desoxyribonukleinsäure (kurz DNA für Englisch "deoxyribonucleic acid"), Plasmide, Impfstoffe, Extrazelluläre Vesikel, Liposome, Sekretome, Gerinnungsfaktoren und Albumin. Bei den extrazellulären Vesikel handelt es sich im Kontext der vorliegenden Anmeldung um Exosome und/oder Mikrovesikel und/oder Apoptosekörperchen. Im Kontext der vorliegenden Anmeldung ist die Verwendung für ein biotechnologisches Produktionsverfahren oder Filtrationsverfahren einer biologischen oder biotechnischen Flüssigkeit, welche ein aus dem Überstand einer Zellkultur gewonnen wird oder ein Überstand einer Zellkultur ist

Vorzugsweise wird erfindungsgemäß eine möglichst hohe Konzentration an aufzureinigenden Stoffen, wie z.B. Vesikeln, d.h. eine möglichst geringe Flüssigkeitsmenge angestrebt. Denkbar ist beispielsweise, dass ein Batch von 9 I erfindungsgemäß auf ein Volumen von ca. 100 ml reduziert wird, was zu einem entsprechenden Anstieg der Dichte bzw. Konzentration der aufzureinigenden Stoffe führt.

Auch für das Substrat, d.h. für die Flüssigkeit kommen andere Flüssigkeiten als Zellüberstände in Betracht, wie z.B. Blutserum oder Blutplasma.

Auch der Einsatz der Erfindung in therapeutischen Verfahren, wie z.B. der Dialyse ist denkbar und von der Erfindung umfasst.

In einer bevorzugten Ausführungsform der erfindungsgemäßen Verwendung eines Filtermoduls für ein biotechnologisches Produktionsverfahren ist das Verfahren ein Filtrationsverfahren.

In einer bevorzugten Ausführungsform der erfindungsgemäßen Verwendung eines Filtermoduls für ein biotechnologisches Produktionsverfahren ist das Verfahren ein Tangentialfluss-Filtrationsverfahren (TFF).

In einer bevorzugten Ausführungsform der erfindungsgemäßen Verwendung eines Filtermoduls für ein biotechnologisches Produktionsverfahren ist das Verfahren ein Verfahren zur Filtration oder Konzentration biologischer Makromoleküle und biologischer Mikrostrukturen, insbesondere extrazellulärer Vesikel, in einer biotechnologischen Flüssigkeit.

In einer bevorzugten Ausführungsform der erfindungsgemäßen Verwendung eines Filtermoduls für ein biotechnologisches Produktionsverfahren ist das Verfahren ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit.

In einer bevorzugten Ausführungsform des erfindungsgemäßen Filtermoduls ist der Zentrifugalpumpenrotor fluidisch stromabwärts oder stromaufwärts auf einer Längsachse des Filters angeordnet. Ein solches Filtermoduls kann gemäß einem Aspekt insbesondere für ein biotechnologisches Filtrationsverfahren, insbesondere ein Tangentialfluss-Filtrationsverfahren (TFF), ein Verfahren zur Filtration oder Konzentration biologischer Makromoleküle und biologischer Mikrostrukturen, insbesondere extrazellulärer Vesikel, in einer biotechnologischen Flüssigkeit oder ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit eingesetzt werden.

In einer bevorzugten Ausführungsform des erfindungsgemäßen Filtermoduls liegt die mit dem Betrieb des Zentrifugalpumpenrotors assoziierte Achse des Zentrifugalpumpenrotors auf einer Längsachse des Filters. Ein solches Filtermodul kann gemäß einem Aspekt insbesondere für ein biotechnologisches Filtrationsverfahren, insbesondere ein Tangentialfluss-Filtrationsverfahren (TFF), ein Verfahren zur Filtration oder Konzentration biologischer Makromoleküle und biologischer Mikrostrukturen, insbesondere extrazellulärer Vesikel, in einer biotechnologischen Flüssigkeit oder ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit eingesetzt werden.

In einer bevorzugten Ausführungsform des erfindungsgemäßen Filtermoduls ist der Filter ein Dialysator für Hämodialyse. Dadurch ergibt sich besonders vorteilhaft eine erhöhte Robustheit des Filters gegenüber Druckunterschieden. Ein solches Filtermodul kann gemäß einem Aspekt insbesondere für ein biotechnologisches Filtrationsverfahren, insbesondere ein Tangentialfluss-Filtrationsverfahren (TFF), ein Verfahren zur Filtration oder Konzentration biologischer Makromoleküle und biologischer Mikrostrukturen, insbesondere extrazellulärer Vesikel, in einer biotechnologischen Flüssigkeit oder ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit eingesetzt werden.

In einer bevorzugten Ausführungsform des erfindungsgemäßen Filtermoduls ist der Zentrifugalpumpenrotor derart mit Permanentmagneten ausgestattet ist, dass er magnetisch levitierend gelagert betrieben und/oder angetrieben werden kann. Aus einer magnetisch levitierenden Lagerung ergibt sich besonders vorteilhaft der Wegfall von mechanischen Lagerungen und damit der Wegfall von Hitzequellen in der Nähe einer geförderten Flüssigkeit. So kann eine potentielle Hitzeschädigung einer geförderten Flüssigkeit vermieden werden. Ein solches Filtermodul kann gemäß einem Aspekt insbesondere für ein biotechnologisches Filtrationsverfahren, insbesondere ein Tangentialfluss-Filtrationsverfahren (TFF), ein Verfahren zur Filtration oder Konzentration biologischer Makromoleküle und biologischer Mikrostrukturen, insbesondere extrazellulärer Vesikel, in einer biotechnologischen Flüssigkeit oder ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit eingesetzt werden.

In einer bevorzugten Ausführungsform des erfindungsgemäßen Filtermoduls weist das Gehäuse eine erste Endkappe auf, die einen Eingangsflusspfad für eine Flüssigkeit L zum Zentrifugalpumpenrotor so definiert, dass eine Flüssigkeit L senkrecht zur Längsachse des Filters in das Gehäuse einströmen und so umgelenkt werden kann, dass sie parallel zur Längsachse den Zentrifugalpumpenrotor anströmt Ein solches Filtermodul kann gemäß einem Aspekt insbesondere für ein biotechnologisches Filtrationsverfahren, insbesondere ein Tangentialfluss-Filtrationsverfahren (TFF), ein Verfahren zur Filtration oder Konzentration biologischer Makromoleküle und biologischer Mikrostrukturen, insbesondere extrazellulärer Vesikel, in einer biotechnologischen Flüssigkeit oder ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit eingesetzt werden.

In einer bevorzugten Ausführungsform des erfindungsgemäßen Filtermoduls weist das Gehäuse eine erste Endkappe auf, die einen Eingangsflusspfad für eine Flüssigkeit L zum Zentrifugalpumpenrotor so definiert, dass eine Flüssigkeit L senkrecht zur Längsachse des Filters in das Gehäuse einströmen und so umgelenkt werden kann, dass sie parallel zur Längsachse den Zentrifugalpumpenrotor anströmt, und der Eingangsflusspfad ist so gestaltet, dass die Flüssigkeit in das Zentrum des Zentrifugalpumpenrotors strömt. Ein solches Filtermodul kann gemäß einem Aspekt insbesondere für ein biotechnologisches Filtrationsverfahren, insbesondere ein Tangentialfluss-Filtrationsverfahren (TFF), ein Verfahren zur Filtration oder Konzentration biologischer Makromoleküle und biologischer Mikrostrukturen, insbesondere extrazellulärer Vesikel, in einer biotechnologischen Flüssigkeit oder ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit eingesetzt werden.

In einer bevorzugten Ausführungsform des erfindungsgemäßen Filtermoduls weist das Gehäuse eine erste Endkappe auf, die einen Eingangsflusspfad für eine Flüssigkeit L zum Zentrifugalpumpenrotor so definiert, dass eine Flüssigkeit senkrecht zur Längsachse des Filters in das Gehäuse einströmen und so umgelenkt werden kann, dass sie parallel zur Längsachse den Zentrifugalpumpenrotor anströmt, und es definieren Zentrifugalpumpenrotor und Endkappe einen Flüssigkeitspfad so, dass Flüssigkeit zentrifugal aus dem Zentrifugalpumpenrotor senkrecht zur Längsachse des Filters herausströmen kann und so umgelenkt wird, dass sie danach parallel zur Längsachse des Filters strömt und in die Fasern des Filters eintritt, wenn der Zentrifugalpumpenrotor rotiert wird. In einer Weiterbildung kann zusätzlich der Eingangsflusspfad so gestaltet sein, dass die Flüssigkeit in das Zentrum des Zentrifugalpumpenrotors strömt. Ein solches Filtermodul kann gemäß einem Aspekt insbesondere für ein biotechnologisches Filtrationsverfahren, insbesondere ein Tangentialfluss-Filtrationsverfahren (TFF), ein Verfahren zur Filtration oder Konzentration biologischer Makromoleküle und biologischer Mikrostrukturen, insbesondere extrazellulärer Vesikel, in einer biotechnologischen Flüssigkeit oder ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit eingesetzt werden.

In einer bevorzugten Ausführungsform weist eine erfindungsgemäße Filtrationsvorrichtung ein erfindungsgemäßes Filtermodul, welches koppelbar ist mit einer Antriebseinheit, und eine Antriebseinheit zum magnetischen Antrieb des Zentrifugalpumpenrotors des Filtermoduls, welche dynamische Magnetfelder erzeugen kann zum magnetischen Antrieb und zur magnetischen Lagerung des Zentrifugalpumpenrotors, wenn das Filtermodul mit dem Antriebsmodul gekoppelt ist, auf. Im Betrieb der Filtrationsvorrichtung ist das Filtermodul gekoppelt mit der Antriebseinheit und die Antriebseinheit erzeugt dynamische Magnetfelder, die den Zentrifugalpumpenrotor des Filtermoduls magnetisch antreiben und magnetisch levitierend lagern. Eine solche Filtrationsvorrichtung kann gemäß einem Aspekt insbesondere für ein biotechnologisches Filtrationsverfahren, insbesondere ein Tangentialfluss-Filtrationsverfahren (TFF), ein Verfahren zur Filtration oder Konzentration biologischer Makromoleküle und biologischer Mikrostrukturen, insbesondere extrazellulärer Vesikel, in einer biotechnologischen Flüssigkeit oder ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit eingesetzt werden.

In einer bevorzugten Ausführungsform einer erfindungsgemäßen Verwendung wird ein erfindungsgemäßes Filtermodul oder einer erfindungsgemäße Filtrationsvorrichtung für ein biotechnologisches Filtrationsverfahren zur Konzentration oder Filtration biologischer Makromoleküle und/oder biologischer Mikrostrukturen in einer biotechnologischen Flüssigkeit verwendet. Bevorzugt handelt es sich bei den biologischen Makromolekülen und/oder biologischen Mikrostrukturen um eine oder mehrere Substanzen aus: Antikörper, Antikörper-Konjugate, Antikörperfragment-Konjugate, Viruspartikel, Ribonukleinsäure (kurz RNA für Englisch "ribonucleic acid"), Desoxyribonukleinsäure (kurz DANN für Englisch "deoxyribonucleic acid"), Plasmide, Impfstoffe, Extrazelluläre Vesikel, Liposome, Sekretome, Gerinnungsfaktoren und Albumin. Bei den extrazellulären Vesikel handelt es sich beispielsweise um Exosome und/oder Mikrovesikel und/oder Apoptosekörperchen.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente.

Weitere Einzelheiten und Vorteile der Erfindung werden anhand eines in der Zeichnung dargestellten Ausführungsbeispiels näher erläutert.

### Kurze Beschreibung der Zeichnungen

Im Folgenden werden die Vorrichtung und das Verfahren mit Bezug auf die Zeichnung beschrieben. In der Zeichnung zeigt:
- Figur 1: ein erfindungsgemäßes Filtermodul zur Verwendung für eine Filtration einer biotechnologischen Flüssigkeit schematisch (in einer ersten Ausführungsform),
- Figur 2: eine Schnittzeichnung durch den Bereich der ersten Endkappe einer beispielhaften Ausführung eines erfindungsgemäßen Filtermoduls,
- Figur 3: dieselbe Anordnung wie Figur 2, jedoch perspektivisch, wobei zusätzlich eine optionale innere Stützplatte mit Öffnungen in der ersten Endkappe erkennbar ist,
- Figur 4: eine beispielhafte Ausführungsform eines erfindungsgemäßen Zentrifugalpumpenrotors,
- Figur 5: den Flussplan einer beispielhaften Ausführungsform einer erfindungsgemäßen Filtrationsvorrichtung, wie sie bei einer erfindungsgemäßen Verwendung eines Filtermoduls aufweisend, in einem Filtergehäuse, einen Filter mit einem Bündel hohler Fasern und einen Zentrifugalpumpenrotor, für ein biotechnologisches ein biotechnologisches Produktionsverfahren, Anwendung findet,
- Figur 6: den Flussplan einer besonderen Ausführungsform einer erfindungsgemäßen Filtrationsvorrichtung, bei der der Filter des Filtermoduls wie ein bekannter Dialysator für Hämodialyse aufgebaut ist, wobei die Filtrationsvorrichtung eine zusätzliche Pumpe stromabwärts des Permeatanschlusses aufweist,
- Figur 7: den Flussplan aus Figur 6 zusätzlich mit einem optionalen Rückschlagventil, das stromabwärts der zusätzlichen Pumpe an dem Anschluss zur Abfuhr von Permeat angeordnet ist.

In den Figuren können gleiche oder ähnliche Elemente mit denselben Bezugszeichen referenziert sein.

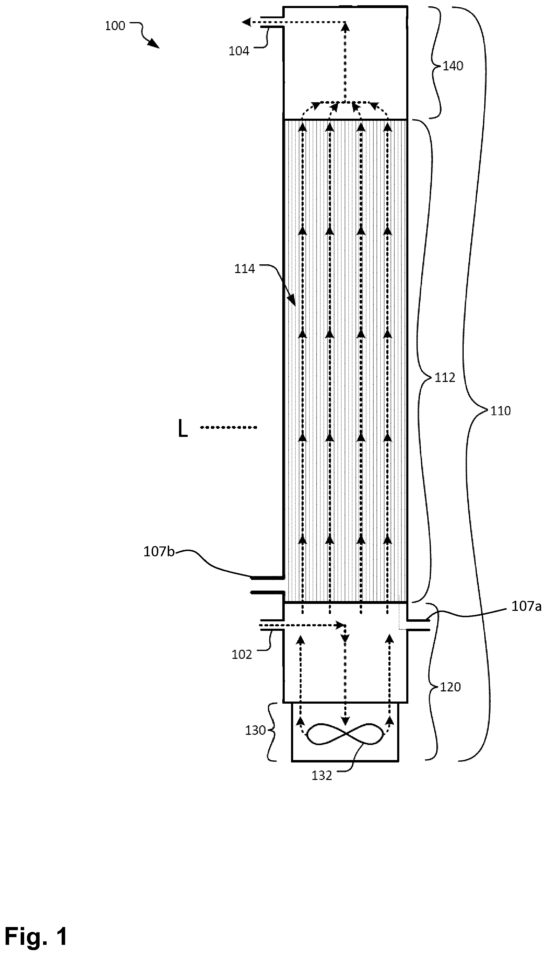

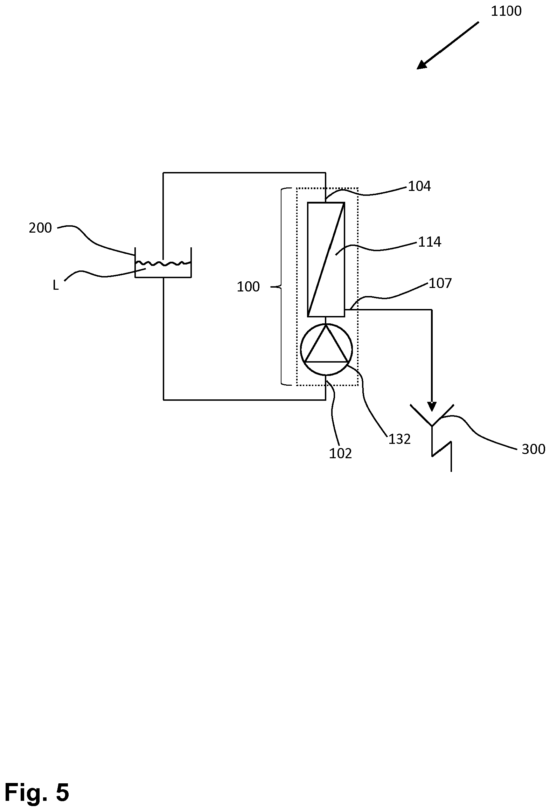

Figur 1 zeigt ein Filtermodul 100 zur Filtration einer biotechnologischen Flüssigkeit L aufweisend ein Gehäuse 110 mit einer ersten Endkappe 120, einer zweiten Endkappe 140 und einem Mittelteil 112 zwischen den Endkappen. Ein Bündel hohler Fasern 114 ist darin so angeordnet, dass es sich mit seiner Längsachse von der ersten Endkappe 120 zur zweiten Endkappe 140 durch den Mittelteil 112 des Gehäuses erstreckt. Die erste Endkappe 120 weist ein Zentrifugalpumpenrotorgehäuse 130 auf, in dem ein Zentrifugalpumpenrotor 132, vorzugsweise ein Impeller, angeordnet ist. An der ersten Endkappe 120 ist ferner ein erster Anschluss 102 angeordnet, welcher der Zufuhr einer zu filtrierenden biotechnologischen Flüssigkeit dient, und fluidisch verbunden ist mit dem Innenraum der hohlen Fasern 114 des Filters. An der zweiten Endkappe 140 ist ein zweiter Anschluss 104 für die Abfuhr einer zu filtrierenden biotechnologischen Flüssigkeit L angeordnet, welcher ebenfalls mit dem Innenraum der hohlen Fasern 114 des Filters fluidisch verbunden ist. Wenn die Vorrichtung zur Filtration einer biotechnologischen Flüssigkeit L verwendet wird, dreht sich der Zentrifugalpumpenrotor 132 und pumpt die biotechnologische Flüssigkeit L, die durch den ersten Anschluss 102 einströmt, in den Hohlraum im Inneren der Fasern 114 des Faserbündels. Ein Teil der Flüssigkeit L tritt durch die Faserwände hinaus in den Außenraum der Fasern. Die durch die Fasern getretene Flüssigkeit, eine üblicherweise als Abfall angesehen Flüssigkeit, das Permeat, kann durch einen dritten Anschluss 107, der am Filtergehäuse 110 angeordnet ist, abgeführt werden. Dieser dritte Anschluss 107 kann an der ersten Endkappe 120 (wie gezeigt als 107a), an der zweiten Endkappe 140 (nicht gezeigt) oder am Mittelteil 112 (wie beispielsweise gezeigt als 107b) angeordnet sein. Figur 1 zeigt zwei Alternativen, wo der dritte Anschluss 107 angeordnet sein kann aber es ist nur einer der gezeigten Anschlüsse erforderlich. Aus der Darstellung von zwei dritten Anschlüssen 107a, 107b kann nicht geschlossen werden, dass dies für die vorliegende Anmeldung erforderlich ist. Vielmehr dient dies der Illustration verschiedener Möglichkeiten. Wesentlich für diesen dritten Anschluss 107 ist, dass er fluidisch verbunden ist mit dem Außenraum der Fasern des Faserbündels 114 des Filters und dass er zugleich durch die Membran getrennt ist vom Innenraum der hohlen Fasern des Faserbündels. In der zweiten Endkappe 140 kann der Teil der biotechnologischen Flüssigkeit, der nicht durch die Faserwand getreten ist, sondern im inneren verblieben ist -das Retentat - wieder aus dem Faserbündel austreten und durch den zweiten Anschluss 104 aus dem Filtermodul abgeführt werden. Typischerweise sind im Verlauf einer biotechnologischen Filtration mehrere Durchläufe einer biotechnologischen Flüssigkeit durch ein Filtermodul 100 erforderlich, um eine gewünschte Konzentration in der biotechnologischen Flüssigkeit L zu erreichen. Daher wird in bekannten biotechnologischen Filtrationsanordnungen häufig ein Kreislauf mit einer Pumpe und einem Reservoir so gebildet, dass die Flüssigkeit mehrmals durch ein Filtermodul gepumpt werden kann. Ein solcher Kreislauf wird nicht in Figur 1, aber in den Figuren 5, 6 und 7 gezeigt.

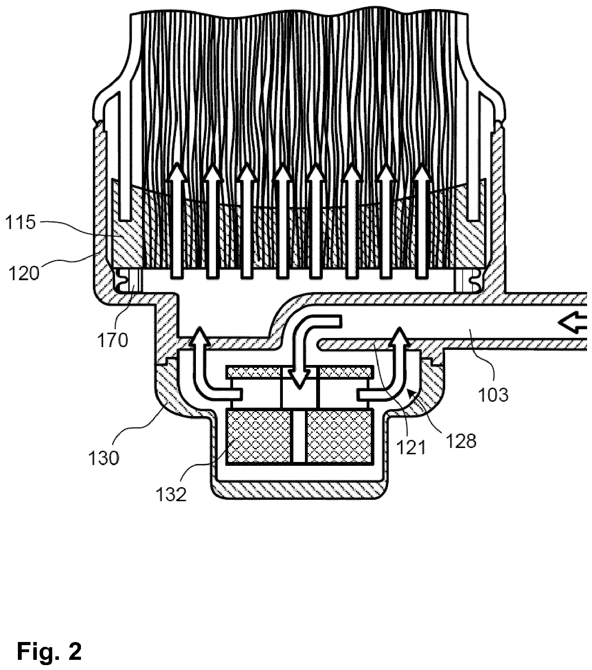

Figur 2 zeigt eine Schnittzeichnung durch den Bereich der ersten Endkappe 120 einer beispielhaften Ausführung eines erfindungsgemäßen Filtermoduls 100. Hierbei soll verdeutlicht werden, wie das Filtermodul 100 im Bereich der ersten Endkappe 120 und des Zentrifugalpumpenrotorgehäuses 130 beispielhaft gestaltet werden kann, sodass ein Eingangsflusspfad für eine Flüssigkeit L gebildet ist, demzufolge die Flüssigkeit L senkrecht zur Längsachse des Filters 114 in das Gehäuse 110 einströmen kann und so umgelenkt werden kann, dass sie danach jedoch parallel zur Längsachse des Bündels hohler Fasern 114 und somit des Filtermoduls 100 den Zentrifugalpumpenrotor 132 anströmen kann. Dabei bezeichnet 115 die Mittel zur Fixierung der Fasern 114 des Faserbündels im Gehäuse 110. Im Fall eines Dialysators für Hämodialyse kann es sich dabei um ein sogenanntes Potting handeln. Weiterhin ist die in Fig. 2 gezeigte Ausführungsform so gestaltet, dass die Flüssigkeit L in das Zentrum des Zentrifugalpumpenrotors 132 einströmen kann. Außerdem ist die in Fig. 2 gezeigte Ausführungsform so gestaltet, dass Zentrifugalpumpenrotor 132, Zentrifugalpumpenrotorgehäuse 130 und Endkappe 120 einen Flüssigkeitspfad so definieren, dass Flüssigkeit L zentrifugal aus dem Zentrifugalpumpenrotor 132 senkrecht zur Längsachse des Filters herausströmen kann und so umgelenkt wird, dass sie stromabwärts des Zentrifugalpumpenrotors 132 und vor Eintritt in die Fasern 114 parallel zur Längsachse des Filters 114, wenn der Zentrifugalpumpenrotor 132 rotiert wird. Im Zusammenspiel ergibt die mechanische Ausgestaltung des Filtergehäuses 110 im Bereich der ersten Endkappe 120, des Zentrifugalpumpenrotors 132 und des Zentrifugalpumpenrotorgehäuses 130 einen Flusspfad, der in einem Abschnitt als koaxial und antiparallel gerichtet bezeichnet werden kann: Das Innere, das hohle Zentrum des Zentrifugalpumpenrotors kann in der Orientierung der Abbildung senkrecht von oben nach unten angeströmt werden, gepumpte Flüssigkeit L wird radial vom Zentrifugalpumpenrotor 132 nach außen befördert, wenn der Zentrifugalpumpenrotor 132 sich dreht, und so umgelenkt, dass sie danach in der Orientierung der Abbildung senkrecht nach oben zu den hohlen Fasern 114 des Faserbündels des Filters strömen kann. Auf der Achse des Zentrifugalpumpenrotors 132 strömt die Flüssigkeit eingangsseitig des Zentrifugalpumpenrotors 132 zentrisch nach unten, koaxial und ringförmig um die Achse des Zentrifugalpumpenrotors 132 strömt die Flüssigkeit ausgangsseitig des Zentrifugalpumpenrotors 132 senkrecht nach oben. Eine vertikale Anordnung von Zentrifugalpumpenrotor und den hohlen Fasern 114 des Faserbündels ergibt den Vorteil, dass in der Flüssigkeit L vorhandene Gasansammlungen durch den Auftrieb und zugleich die von dem Zentrifugalpumpenrotor 132 der Pumpe erzeugte Flüssigkeitsströmung nach oben abtransportiert werden. Die oben beschriebene, koaxiale Anordnung von Zentrifugalpumpenrotor 132 und Faserbündel ermöglicht eine besonders symmetrische Strömung einer Flüssigkeit L durch den Zentrifugalpumpenrotor 132 in das Innere der hohlen Fasern 114 des Faserbündels. Wie aus Figur 2 ersichtlich wird, existieren abschnittsweise Antiparallele, koaxiale Flüssigkeitsströme in einer solchen Situation. Die Flüssigkeit strömt den Zentrifugalpumpenrotor auf der Längsachse des Faserbündels, welche mit der Drehachse des Zentrifugalpumpenrotors zusammenfällt, an und wird nach Verlassen des Zentrifugalpumpenrotors so umgelenkt, dass sie parallel jedoch entgegengesetzt gerichtet zum Faserbündel strömt. Bevorzugt ist das Faserbündel makroskopisch genähert zylinderförmig. Durch diese symmetrische Strömungslage wird der Zentrifugalpumpenrotor hydraulisch stabilisiert. Das minimiert ein potentielles Flattern des Zentrifugalpumpenrotors. Ein Flattern des Zentrifugalpumpenrotors könnte zu einer Schädigung der biotechnologischen Flüssigkeit führen. Somit wird auch eine mögliche Schädigung der biotechnologischen Flüssigkeit vermieden. Besonders bei magnetisch levitierend gelagerten Zentrifugalpumpenrotoren ergibt sich weiterhin der Vorteil, dass weniger exzentrische Lagermomente auftreten oder die exzentrischen Lagermomente sind weniger stark. Dadurch reichen weniger starke Magnetfelder zum Antrieb und zur Lagerung des Zentrifugalpumpenrotors aus. Die weniger starken Magnetfelder ermöglichen wiederum, weniger Energie und/oder geringere elektrische Ströme im Erzeuger der magnetischen Wechselfelder der Antriebseinheit zu benötigen. Zur verbesserten Strömungsführung in dem im Wesentlichen ringförmigen Ausströmungsbereich 128 des Zentrifugalpumpenrotors 132 kann eine optionale innere Stützplatte 121 in der ersten Endkappe 120 vorgesehen sein. Zwischen dem Faserbündel hohler Fasern 114 und der Wand der ersten Endkappe 120 bzw. dem optionalen Potting 115 kann eine optionale Dichtung 170 vorgesehen sein.

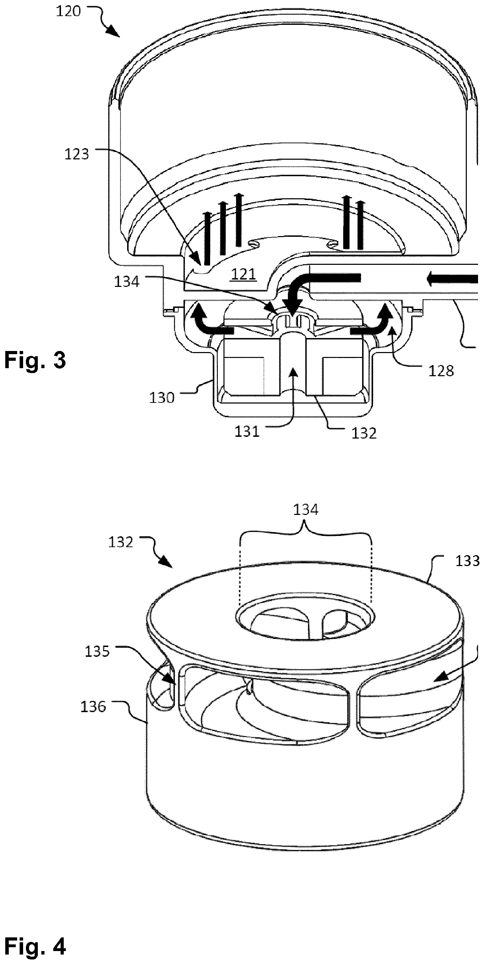

Figur 3 zeigt dieselbe Anordnung wie Figur 2, jedoch perspektivisch. Zusätzlich sind darin Öffnungen 123 in einer optionale innere Stützplatte 121 in der ersten Endkappe 120 erkennbar, die zwischen dem Zentrifugalpumpenrotor 132 und dem Filterbündel 114 so angeordnet sind, dass Flüssigkeit L vom Zentrifugalpumpenrotor 132 durch die Öffnungen 123 und dann zu den 114 Fasern des Filters strömt, wenn der 132 Zentrifugalpumpenrotor in Drehung versetzt ist. Pfeile verdeutlichen die Strömung einer biotechnologischen Flüssigkeit L, wenn der Zentrifugalpumpenrotor sich dreht. So wird der Zentrifugalpumpenrotor 132 beispielsweise so angeströmt, dass die Flüssigkeit durch eine zentrale Öffnung 134 in einer Scheibe 133 in einen zentralen Hohlraum des Zentrifugalpumpenrotors 131 auf dessen Rotationsachse zugeführt wird.

Figur 4 zeigt eine beispielhafte Ausführungsform eines erfindungsgemäßen Zentrifugalpumpenrotors. Hierbei handelt es sich um einen Impeller-Rotor mit hohlem Zentrum. Hierbei wird illustriert, dass die Schaufelblätter 135 des Zentrifugalpumpenrotors beispielsweise gebogen sein können, beispielsweise spiralförmig. Die Schaufelblätter sind zwischen zwei Scheiben angeordnet, wobei die in der Darstellung untere Scheibe an die im Zentrifugalpumpenrotor angeordneten Permanentmagnete 136 angrenzt und die obere Scheibe 133 eine zentrale Öffnung 134 aufweist, durch die zu pumpende Flüssigkeit ins hohle Zentrum des Zentrifugalpumpenrotors 131 einströmen kann.

Fig. 5 zeigt den Flussplan einer Ausführungsform einer erfindungsgemäßen Filtrationsvorrichtung 1100 schematisch. Außer dem Filtermodul 100 kommen dabei beispielsweise ein Abfluss 300 und ein Reservoir 200 für die Flüssigkeit L vor. Die Filtrationsanordnung 1100 ist als Kreislauf der zu filtrierenden bzw. reinigenden biotechnologischen Flüssigkeit L angelegt. In dieser Figur wird eine biotechnologische Filtrationsanordnung 1100, gebildet als ein Kreislauf mit einer Pumpe mit einem Zentrifugalpumpenrotor 132 und einem Reservoir für die im Kreislauf umlaufende biotechnologische Flüssigkeit L, gezeigt, worin eine zu filtrierende Flüssigkeit L mehrmals durch ein Filtermodul 100 gepumpt werden kann. Durch die gepunktete Linie ist angedeutet, dass der Zentrifugalpumprotor und der Filter mit dem Bündel hohler Fasern 114 in einem gemeinsamen Filtergehäuse 110 angeordnet sind. Nicht gezeigt ist die Antriebseinheit, mit der das Filtermodul 100 koppelbar ist und das dynamische Magnetfelder erzeugen kann, die den Zentrifugalpumpenrotor 132, da er Permanentmagnete aufweist, magnetisch antreiben und/oder magnetisch levitierend lagern kann.

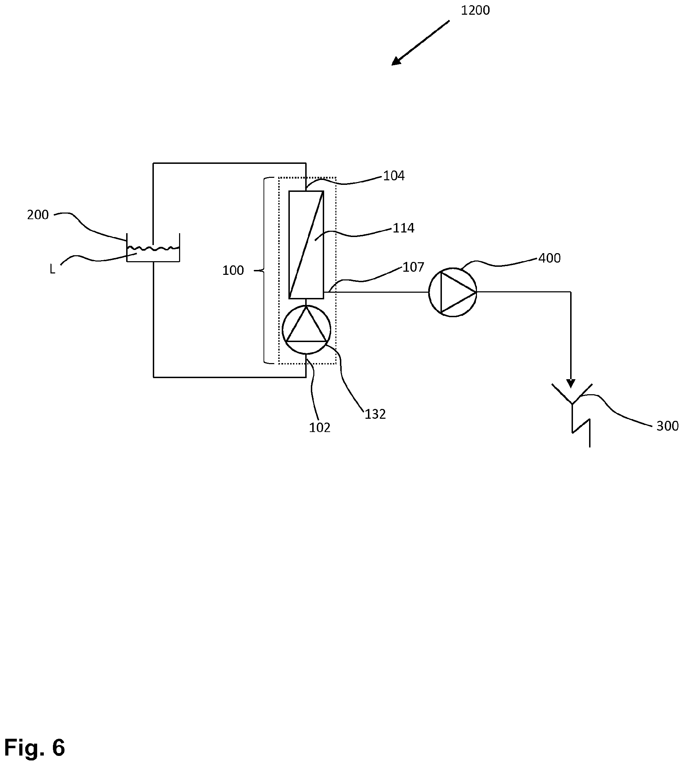

Fig. 6 zeigt den Flussplan einer besonderen Ausführungsform einer erfindungsgemäßen Filtrationsvorrichtung 1200 schematisch. Hierbei ist der Filter des Filtermoduls 100 aufgebaut wie ein bekannter Dialysator für Hämodialyse. Im Vergleich zu Filtern, die üblicherweise in biotechnologischen Filtrationsvorrichtungen eingesetzt werden weist ein Dialysator besonders vorteilhaft eine gegen Druckunterschiede stabilere Faseranordnung auf: Die Fasern 114 haben im Verhältnis zum Außendurchmessern einen kleineren Faserinnendurchmesser. Dadurch ergibt sich die erhöhte Stabilität der Hohlfasermembrane. Zusätzlich sind die Fasern 114 eines Dialysators typischerweise kürzer, was auch in erhöhter Stabilität resultiert. Um die kürzeren Fasern 114 und die dickeren Membranwände zu kompensieren, was die Ausbeute der Filtration angeht, weist ein Dialysator eine höhere Anzahl von Fasern 114 auf. Durch die Verwendung eines Dialysators als Filter im Filtermodul 100 ergibt sich aus der erhöhten Stabilität gegen Druckunterschiede innerhalb / außerhalb der Faser 114, dass man eine zusätzliche Pumpe 400 so anordnen kann, dass sie an den Anschluss 107 zur Abfuhr von abgeschiedener Abfallflüssigkeit, welche fluidisch verbunden ist mit dem Außenraum der hohlen Fasern 114, angeschlossen ist. Die Pumprichtung ist so gewählt, dass das Permeat aus dem Außenraum der Fasern 114 abgesaugt wird. Eine derartige Anordnung würde im Betrieb der zusätzlichen Pumpe 400 bei bekannten Filtrationsvorrichtungen zur Beschädigung der Membrane führen. Besonders vorteilhaft ermöglicht die Anordnung dieser zusätzlichen Pumpe 400 am Anschluss 107 zur Permeatabfuhr, dass sich mit weniger Durchläufen der zu konzentrierenden biotechnologischen Flüssigkeit L eine bestimmte Konzentration erreichen lässt als ohne zusätzliche Pumpe 400. Einerseits wird dadurch besonders vorteilhaft der Zeitbedarf für das Erreichen einer bestimmten Konzentration verkürzt. Andererseits wird dadurch besonders vorteilhaft erreicht, dass die biotechnologische Flüssigkeit L weniger Filterdurchläufen ausgesetzt ist und dadurch weniger Schädigung erfährt. Je häufiger eine biotechnologische Flüssigkeit einen Filter durchläuft, desto höher ist eine potentielle Schädigung. Weiterhin vorteilhaft an dieser Anordnung ist, dass durch die zusätzliche Pumpe 400 keine zusätzliche Schädigung der zu konzentrierenden biotechnologischen Flüssigkeit L entsteht: Die zusätzliche Pumpe 400 kommt nur in Kontakt mit dem Permeat, das ein Abfallprodukt ist.

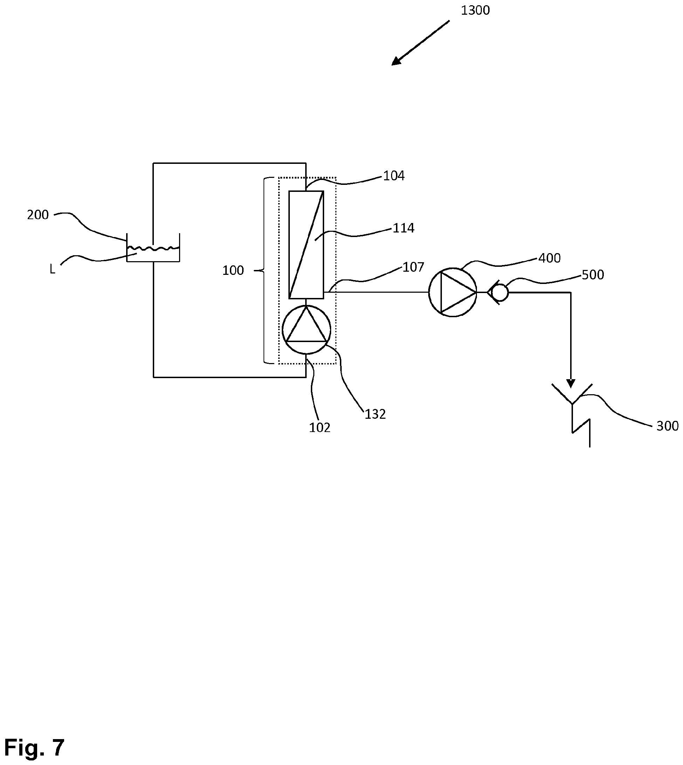

Fig. 7 zeigt den Flussplan einer Filtrationsvorrichtung 1300 aus Figur 6 zusätzlich mit einem optionalen Rückschlagventil 500, dass stromabwärts der zusätzlichen Pumpe 400 an dem Anschluss 107 zur Abfuhr von Permeat angeordnet ist. Das Rückschlagventil 500 befindet sich an der Permeatleitung zwischen der zusätzlichen Pumpe 400 und dem Abfluss 300. Durch dieses optionale, zusätzliche Rückschlagventil 500 lassen sich besonders vorteilhaft in Zusammenspiel mit der zusätzlichen Pumpe 400 die Druckverhältnisse an der Membran des Filters einstellen. Hierzu wird ein Rückschlagventil 500 verwendet, das bei Beaufschlagung eines passend ausgewählten Mindestdrucks von der zusätzlichen Pumpe 400 her öffnet.

## Patentansprüche

1. Filtermodul (100) zur Filtration einer biotechnologischen Flüssigkeit (L) aufweisend
a. ein Filtergehäuse (110),
b. einen in dem Filtergehäuse (110) angeordneten Filter mit einem Bündel von Hohlfasern (114), wobei die Faserwand die Filtermembran bildet,
c. einen Zentrifugalpumpenrotor (132) so angeordnet in dem Filtergehäuse (110), dass er fluidisch verbunden ist mit dem Innenraum der Hohlfasern (114), wobei der Zentrifugalpumpenrotor (132) magnetisch antreibbar ist, sodass er eine Flüssigkeit (L) durch die Hohlfasern (114) drücken kann,
d. einen ersten Anschluss (102) für die Zufuhr einer zu filtrierenden biotechnologischen Flüssigkeit (L), welcher fluidisch verbunden ist mit dem Innenraum der Hohlfasern (114) des Filters,
e. einen zweiten Anschluss (104) für die Abfuhr einer zu filtrierenden biotechnologischen Flüssigkeit (L), welcher fluidisch verbunden ist mit dem Innenraum der Hohlfasern (114) des Filters,
f. einen dritten Anschluss (107) zur Abfuhr von abgeschiedener Abfallflüssigkeit, welche fluidisch verbunden ist mit dem Außenraum der Hohlfasern (114).

2. Filtermodul (100) nach Anspruch 1, wobei das Bündel einer zylindrischen Anordnung von Hohlfasern entspricht und der Zentrifugalpumpenrotor (132) fluidisch stromabwärts oder stromaufwärts auf der Längsachse des Filters angeordnet ist, die der Rotationsachse des zylindrischen Bündels entspricht.

3. Filtermodul (100) nach Anspruch 2, wobei die mit dem Betrieb des Zentrifugalpumpenrotors (132) assoziierte Achse des Zentrifugalpumpenrotors (132) auf der Längsachse des Filters liegt.

4. Filtermodul (100) nach einem der Ansprüche 1 bis 3, wobei der Filter ein Dialysator für Hämodialyse ist.

5. Filtermodul (100) nach einem der Ansprüche 1 bis 4, wobei der Zentrifugalpumpenrotor (132) derart mit Permanentmagneten ausgestattet ist, dass er magnetisch levitierend gelagert betrieben und/oder angetrieben werden kann.

6. Filtermodul (100) nach einem der Ansprüche 2 bis 5, wobei das Gehäuse (110) eine erste Endkappe (120) aufweist, die einen Eingangsflusspfad für eine Flüssigkeit (L) zum Zentrifugalpumpenrotor (132) so definiert, dass eine Flüssigkeit (L) senkrecht zur Längsachse des Filters in das Gehäuse (110) einströmen und so umgelenkt werden kann, dass sie parallel zur Längsachse den Zentrifugalpumpenrotor (132) anströmt.

7. Filtermodul (100) nach Anspruch 6, wobei der Eingangsflusspfad so gestaltet ist, dass die Flüssigkeit (L) in das hohle Zentrum (131) des Zentrifugalpumpenrotors (132) strömt.

8. Filtermodul (100) nach einem der Ansprüche 6 oder 7, wobei Zentrifugalpumpenrotor (132) und Endkappe (120) einen Flüssigkeitspfad so definieren, dass Flüssigkeit (L) zentrifugal aus dem Zentrifugalpumpenrotor (132) senkrecht zur Längsachse des Filters herausströmen kann und so umgelenkt wird, dass sie danach parallel zur Längsachse des Filters strömt und in die Hohlfasern (114) des Filters, wobei die Faserwand die Filtermembran bildet, eintritt, wenn der Zentrifugalpumpenrotor (132) rotiert wird.

9. Filtrationsvorrichtung (1100, 1200, 1300) aufweisend
a. ein Filtermodul (100) nach einem der Ansprüche 1 bis 8, welches koppelbar ist mit einer Antriebseinheit, und
b. eine Antriebseinheit zum magnetischen Antrieb des Zentrifugalpumpenrotors (132) des Filtermoduls (100), welche dynamische Magnetfelder erzeugen kann zum magnetischen Antrieb und zur magnetischen Lagerung des Zentrifugalpumpenrotors (132), wenn das Filtermodul (100) mit dem Antriebsmodul gekoppelt ist.

10. Verwendung eines Filtermoduls (100) nach einem der Ansprüche 1 bis 8 oder einer Filtrationsvorrichtung (1100, 1200, 13000) nach Anspruch 9 für ein biotechnologisches Filtrationsverfahren zur Konzentration oder Filtration biologischer Makromoleküle und/oder biologischer Mikrostrukturen in einer biotechnologischen Flüssigkeit (L).

11. Verwendung eines Filtermoduls (100) nach Anspruch 1 aufweisend, in einem Filtergehäuse (110), einen Filter mit einem Bündel Hohlfasern (114), wobei die Faserwand die Filtermembran bildet, und einen Zentrifugalpumpenrotor (132), für ein biotechnologisches Produktionsverfahren.

12. Verwendung eines Filtermoduls (100) für ein biotechnologisches Produktionsverfahren nach Anspruch 11, wobei das Verfahren ein Filtrationsverfahren ist.

13. Verwendung eines Filtermoduls (100) für ein biotechnologisches Produktionsverfahren nach Anspruch 12, wobei das Verfahren ein Tangentialfluss-Filtrationsverfahren (TFF) ist.

14. Verwendung eines Filtermoduls (100) für ein biotechnologisches Produktionsverfahren nach Anspruch 13, wobei das Verfahren ein Verfahren zur Filtration oder Konzentration biologischer Makromoleküle und biologischer Mikrostrukturen, insbesondere extrazellulärer Vesikel, in einer biotechnologischen Flüssigkeit (L) ist.

15. Verwendung eines Filtermoduls (100) für ein biotechnologisches Produktionsverfahren nach Anspruch 14, wobei das Verfahren ein Tangentialfluss-Filtrationsverfahren (TFF) zur Filtration oder Konzentration biologischer Makromoleküle und biologischer Mikrostrukturen, insbesondere extrazellulärer Vesikel, in einer biotechnologischen Flüssigkeit (L) ist.

## Claims

1. Filter module (100) for the filtration of a biotechnological liquid (L), having
a. a filter housing (110),
b. a filter arranged in the filter housing (110) with a bundle of hollow fibres (114), wherein the fibre wall forms the filter membrane,
c. a centrifugal pump rotor (132) arranged in the filter housing (110) such that it is fluidically connected to the interior of the hollow fibres (114), wherein the centrifugal pump rotor (132) is able to be magnetically driven such that it can force a liquid (L) through the hollow fibres (114),
d. a first port (102) for the supply of a biotechnological liquid (L) to be filtered, which first port (102) is fluidically connected to the interior of the hollow fibres (114) of the filter,
e. a second port (104) for the removal of a biotechnological liquid (L) to be filtered, which second port (104) is fluidically connected to the interior of the hollow fibres (114) of the filter,
f. a third port (107) for the removal of precipitated waste liquid, which third port (107) is fluidically connected to the exterior of the hollow fibres (114).

2. Filter module (100) according to Claim 1, wherein the bundle corresponds to a cylindrical arrangement of hollow fibres, and the centrifugal pump rotor (132) is arranged fluidically upstream or downstream on the longitudinal axis of the filter that corresponds to the axis of rotation of the cylindrical bundle.

3. Filter module (100) according to Claim 2, wherein the axis of the centrifugal pump rotor (132) associated with the operation of the centrifugal pump rotor (132) lies on the longitudinal axis of the filter.

4. Filter module (100) according to one of Claims 1 to 3, wherein the filter is a dialyser for haemodialysis.

5. Filter module (100) according to one of Claims 1 to 4, wherein the centrifugal pump rotor (132) is equipped with permanent magnets in such a way that it can be operated and/or driven in a manner supported by magnetic levitation.

6. Filter module (100) according to one of Claims 2 to 5, wherein the housing (110) has a first end cap (120), which defines an inlet flow path for a liquid (L) to the centrifugal pump rotor (132) such that a liquid (L) can flow into the housing (110) perpendicular to the longitudinal axis of the filter and can be deflected such that it flows onto the centrifugal pump rotor (132) parallel to the longitudinal axis.

7. Filter module (100) according to Claim 6, wherein the inlet flow path is designed such that the liquid (L) flows into the hollow centre (131) of the centrifugal pump rotor (132).

8. Filter module (100) according to either of Claims 6 and 7, wherein centrifugal pump rotor (132) and end cap (120) define a liquid path such that liquid (L) can flow centrifugally out of the centrifugal pump rotor (132) perpendicular to the longitudinal axis of the filter and is deflected such that it thereafter flows parallel to the longitudinal axis of the filter and enters the hollow fibres (114) of the filter, the fibre wall forming the filter membrane, when the centrifugal pump rotor (132) is rotated.

9. Filtration device (1100, 1200, 1300), having
a. a filter module (100) according to one of Claims 1 to 8, which is able to be coupled to a drive unit, and
b. a drive unit for magnetically driving the centrifugal pump rotor (132) of the filter module (100), which drive unit can generate dynamic magnetic fields for magnetically driving and magnetically supporting the centrifugal pump rotor (132) when the filter module (100) is coupled to the drive module.

10. Use of a filter module (100) according to one of Claims 1 to 8 or of a filtration device (1100, 1200, 1300) according to Claim 9 for a biotechnological filtration method for the concentration or filtration of biological macromolecules and/or biological microstructures in a biotechnological liquid (L).

11. Use of a filter module (100) according to Claim 1 having, in a filter housing (110), a filter with a bundle of hollow fibres (114), the fibre wall forming the filter membrane, and a centrifugal pump rotor (132), for a biotechnological production method.

12. Use of a filter module (100) for a biotechnological production method according to Claim 11, wherein the method is a filtration method.

13. Use of a filter module (100) for a biotechnological production method according to Claim 12, wherein the method is a tangential flow filtration (TFF) method.

14. Use of a filter module (100) for a biotechnological production method according to Claim 13, wherein the method is a method for the filtration or concentration of biological macromolecules and biological microstructures, in particular extracellular vesicles, in a biotechnological liquid (L).

15. Use of a filter module (100) for a biotechnological production method according to Claim 14, wherein the method is a tangential flow filtration (TFF) method for the filtration or concentration of biological macromolecules and biological microstructures, in particular extracellular vesicles, in a biotechnological liquid (L).

## Revendications

1. Module de filtration (100) destiné à filtrer un liquide biotechnologique (L), ledit module de filtration comportant
a. un boîtier de filtre (110),
b. un filtre disposé dans le boîtier de filtre (110) et comprenant un faisceau de fibres creuses (114), la paroi de fibre formant la membrane de filtration,
c. un rotor de pompe centrifuge (132) disposé dans le boîtier de filtre (110) de manière à être relié fluidiquement à l'espace intérieur des fibres creuses (114), le rotor de pompe centrifuge (132) pouvant être entraîné magnétiquement de sorte qu'il presse un liquide (L) à travers les fibres creuses (114),
d. un premier raccord (102) qui est destiné à amener un liquide biotechnologique (L) à filtrer et qui est relié fluidiquement à l'espace intérieur des fibres creuses (114) du filtre,
e. un deuxième raccord (104) qui est destiné à évacuer un liquide biotechnologique (L) à filtrer et qui est relié fluidiquement à l'espace intérieur des fibres creuses (114) du filtre,
f) un troisième raccord (107) qui est destiné à évacuer un liquide de rejet séparé et qui est relié fluidiquement à l'espace extérieur des fibres creuses (114) .

2. Module de filtration (100) selon la revendication 1, le faisceau correspondant à un ensemble cylindrique de fibres creuses et le rotor de pompe centrifuge (132) étant disposé fluidiquement en aval ou en amont de l'axe longitudinal du filtre qui correspond à l'axe de rotation du faisceau cylindrique.

3. Module de filtration (100) selon la revendication 2, l'axe du rotor de pompe centrifuge (132), associé au fonctionnement du rotor de pompe centrifuge (132), étant situé sur l'axe longitudinal du filtre.

4. Module de filtration (100) selon l'une des revendications 1 à 3, le filtre étant un dialyseur destiné à l'hémodialyse.

5. Module de filtration (100) selon l'une des revendications 1 à 4, le rotor de pompe centrifuge (132) étant équipé d'aimants permanents de manière à pouvoir être actionné et/ou entraîné par lévitation magnétique.

6. Module de filtration (100) selon l'une des revendications 2 à 5, le boîtier (110) comportant un premier capuchon d'extrémité (120) qui définit un chemin d'écoulement d'entrée destiné à un liquide (L) vers le rotor de pompe centrifuge (132) de sorte qu'un liquide (L) puisse s'écouler jusque dans le boîtier (110) perpendiculairement à l'axe longitudinal du filtre et puisse être dévié de manière à affluer vers le rotor de la pompe centrifuge (132) parallèlement à l'axe longitudinal.

7. Module de filtration (100) selon la revendication 6, le chemin d'écoulement d'entrée étant conçu de telle sorte que le liquide (L) s'écoule jusque dans le centre creux (131) du rotor de pompe centrifuge (132).

8. Module de filtration (100) selon l'une des revendications 6 ou 7, le rotor de pompe centrifuge (132) et le capuchon d'extrémité (120) définissant un chemin de liquide de telle sorte que le liquide (L) puisse s'écouler par centrifugation hors du rotor de pompe centrifuge (132) perpendiculairement à l'axe longitudinal du filtre et soit dévié de sorte qu'il s'écoule ensuite parallèlement à l'axe longitudinal du filtre et entre dans les fibres creuses (114) du filtre lorsque le rotor de pompe centrifuge (132) tourne, la paroi de fibre formant la membrane de filtration.

9. Dispositif de filtration (1100, 1200, 1300) comportant
a. un module de filtration (100) selon l'une des revendications 1 à 8, qui peut être accouplé à une unité d'entraînement, et
b. une unité d'entraînement qui est destinée à entraîner magnétiquement le rotor de pompe centrifuge (132) du module de filtration (100) et qui peut générer des champs magnétiques dynamiques afin d'entraîner magnétiquement et supporter magnétiquement le rotor de pompe centrifuge (132) lorsque le module de filtration (100) est accouplé au module d'entraînement.

10. Utilisation d'un module de filtration (100) selon l'une des revendications 1 à 8 ou d'un dispositif de filtration (1100, 1200, 13000) selon la revendication 9 pour mettre en œuvre un procédé de filtration biotechnologique destiné à la concentration ou à la filtration de macromolécules biologiques et/ou de microstructures biologiques dans un liquide biotechnologique (L).

11. Utilisation d'un module de filtration (100) selon la revendication 1, comportant, dans un boîtier de filtre (110), un filtre à faisceau de fibres creuses (114), la paroi de fibre formant la membrane de filtration, ainsi qu'un rotor de pompe centrifuge (132), dans un procédé de production biotechnologique.

12. Utilisation d'un module de filtration (100) dans un procédé de production biotechnologique selon la revendication 11, le procédé étant un procédé de filtration.

13. Utilisation d'un module de filtration (100) dans un procédé de production biotechnologique selon la revendication 12, le procédé étant un procédé de filtration à flux tangentiel (TFF).

14. Utilisation d'un module de filtration (100) dans un procédé de production biotechnologique selon la revendication 13, le procédé étant un procédé de filtration ou de concentration de macromolécules biologiques et de microstructures biologiques, notamment de vésicules extracellulaires, dans un liquide biotechnologique (L).

15. Utilisation d'un module de filtration (100) dans un procédé de production biotechnologique selon la revendication 14, le procédé étant un procédé de filtration à flux tangentiel (TFF) destiné à la filtration ou à la concentration de macromolécules biologiques et de microstructures biologiques, en particulier de vésicules extracellulaires, dans un liquide biotechnologique (L).
